# EUROPEAN PATENT SPECIFICATION

(11) **EP 1 295 948 B1**
(45) Date of publication and mention of the grant of the patent: **20.08.2008**
(21) Application number: 02026802.5
(22) Date of filing: 27.02.1997
(51) Int. Cl.: C12Q 1/37

(54) **Method of measurement of protease and thin membranes used for said method**
Verfahren zur Analyse von Proteasen und dünner Membran zur Verwendung dieses Verfahrens
Procédé d'analyse de protéases et membrane fine utilisée dans ce procédé

(30) Priority: 29.02.1996 JP 4264696
(43) Date of publication of application: 26.03.2003
(62) Divisional of application: 97905416.0
(73) Proprietor: FUJIFILM Corporation, Minato-ku Tokyo (JP)
(72) Inventor: Ogawa, Masashi, c/o Fuji Photo Film Co., Ltd., Minami-ashigara-shi, Kanagawa 250-01 (JP); Hamaoka, Tsutomu, c/o Fuji Photo Film Co., Ltd., Tokyo 106 (JP); Tamura, Yutaka, c/o Fuji Photo Film Co., Ltd., Minami-ashigara-shi, Kanagawa 250-01 (JP); Tachikawa, Tetsuhiko, Tokyo 142 (JP); Hasegawa, Ikuo, Tokyo 160 (JP); Yoshiki, Shusaku, Kawasaki-shi, Kanagawa 216 (JP); Hasegawa, Kohji, Tokyo 160 (JP); Nishigaki, Junji, c/o Fuji Photo Film Co., Ltd., Minami-ashigara-shi, Kanagawa 250-01 (JP)
(74) Representative: HOFFMANN EITLE

(56) References cited:
- EP-A- 0 176 246
- EP-A- 0 319 334
- US-A- 4 137 082
- DATABASE WPI Derwent Publications Ltd., London, GB; AN 1986-087245 XP002308492 & SU 1 178 761 A (PLANT ROTECTION INS) 15 September 1985 (1985-09-15)
- KAMIYAMA MIKIO ET AL: "ANALYTICAL ELEMENT" WPI WORLD PATENT INFORMATION DERWENT, DERWENT, GB, vol. 1993, no. 33 PA - KONISHIROKU PHOTO IND PN - JP5176795 A, 1993, XP002140997
- JOHNSON-WINT B: "QUANTITATIVE COLLAGEN FILM COLLAGENASE ASSAY FOR LARGE NUMBERS OF SAMPLES" ANALYTICAL BIOCHEMISTRY, ACADEMIC PRESS, NEW YORK, NY, US, vol. 104, no. 1, 1980, pages 175-181, XP000918624 ISSN: 0003-2697
- LEVENSON R: "COLLAGEN FILM MICROASSAY FOR TISSUE COLLAGENASE" ANALYTICAL BIOCHEMISTRY, ACADEMIC PRESS, NEW YORK, NY, US, vol. 76, 1976, pages 579-588, XP000921274 ISSN: 0003-2697

## Description

### Technical Field

The present invention relates to a method for measuring protease. More specifically, it relates to a method for measuring protease which enables accurate diagnosis of malignancy of cancer cells such as infiltrative and metastatic activity, degree of progress of periodontal diseases such as alveolar pyorrhea, destructive pathological conditions in rheumatoid arthritis and the like.

### Related Art

Presence or absence of infiltration into interstitium connective tissue is one of the factors that determine difference of benignancy and malignancy of tumor. In order to clarify the pathological conditions, it is necessary to observe changes of growth dynamics of tumor cells, per se, and to find factors affecting the interaction between tumor cells and interstitium connective tissues. In particular, it has been revealed that proteases are involved in infiltration and metastasis of tumor cells, and accordingly, the inhibition of infiltration and metastasis of malignant tumor cells is expected by controlling proteases. Among such proteases (extracellular matrix lyases), important roles of matrix metalloproteinase (MMP), in particular, has been elucidated in the growth and infiltration of cancer cells, and arterialization (see "Molecular Mechanism of Tumor Metastasis", Ed. By T. Tsuruo, Chapter 8; K. Miyazaki, "Matrix Proteases and Infiltration/metastasis of Cancer", pp.92-107, Medical View Co., Ltd., 1993).

Periodontal diseases progress with destruction of crevicular epithelium and connective tissues which mainly consist of collagen as early nidus, and the involvement of matrix metalloproteinase has also been known in the destruction of tissues (for the involvement of proteases in the destruction of periodontal tissues and correlation of pathological conditions and proteases, see "Science of Periodontal Treatment", Ed. by M. Aono, Chapter VII, M. Shirakawa, "Pathology of Periodontal Tissues", pp.99-109, Ishiyaku Shuppan; Hasegawa et al., "Gelatinase Activity in Gingival Crevicular Fluids (GCF) of Periodontal Disease Patients", Presentation No. A-44, 37th Fall Congress of Japan Periodontal Disease Society and other).

The matrix metalloproteinases degrade extracellular substrates such as collagen, proteoglycan, laminin, fibronectin and gelatin, and the existence of eight types, e.g., MMP-1, 2, 3, 7, 9, 10, are known. Interstitial collagenase (MMP-1), a matrix metalloproteinase known for the longest, distributes over fibroblast, cartilage and other, and cleaves interstitial collagen into 1/4 and 1/3 portions. In periodontal diseases, mainly MMP-2 (gelatinase A) and MMP-9 (gelatinase B) destroy, for example, collagen type IV, laminin, fibronectin, and proteoglycan as components of periodontal tissues. The secretion of matrix metalloproteinase is strongly promoted by EGF and TGF - β as cell growth factors, and the secretion and the expression of activity are controlled by endogenous inhibitors in tissues. However, the ways of suppression of its expression have not been fully clarified when growth factors are involved.

Plasminogen activator (PA), a one of serine proteases, is another protease involved in infiltration and metastasis of tumor cells. The plasminogen activator converts plasminogen into plasmin, and plasmin formed by the action of plasminogen activator converts a prometalloproteinase into a metalloproteinase as an active form. Accordingly, it is considered that infiltration and metastasis of cancer cells are progressed or accelerated by a cascade formed between matrix metalloproteinases and plasminogen activator.

Protease may participate in destructive pathological conditions such as destruction of bone tissue and alveolar periosteum caused by alveolar pyorrhea and destruction of periost and bone tissue caused by rheumatoid arthritis, in addition to the infiltration and metastasis of cancer cells and progress of periodontal diseases (for involvement of proteases in rheumatism, see Nippon Rinsho [Japan Clinic], 50(3), pp.463-467, 1992). Therefore, the measurement of protease in cells and tissues may enable accurate diagnosis of malignancy of cancer cells on the basis of infiltrative and metastatic activity, pathological condition of periodontal diseases, and degree of progress of destructive pathological conditions such as rheumatism (for correlation between levels of infiltration of cancer cells and protease activity, see, for example, Yamagata, et al., Cancer Lett., 59, 51, 1991; Azzam, et al., J. Natl., Cancer Inst., 85, 1758, 1993; Brown, et al., Clin. Exp. Metastasis, 11, 183, 1993; Davies, et al., Br. J. Cancer, 67, 1126, 1993).

As methods for measuring protease, zymography methods where enzyme activity is determined based on the degree of substrate degradation, immunoblotting methods utilizing antibodies specific for each protease or other have been used. For example, a method is known which comprises the steps of homogenizing cancer cells or periodontosis cells, subjecting the extract to electrophoresis utilizing gelatin-containing SDS-polyacrylamide gel, staining the gel after the electrophoresis using Amido Black, followed by determining a sample as protease positive that provides a white clear band not stained. However, according to the method, the preparation of SDS-polyacrylamide gel is required for every measurement and the process takes about 30 hours before obtaining measuring results.

Another method is available where a gel after an electrophoresis according to the SDS-PAGE technique is placed on a membrane, and after blotting procedure, enzymes are detected by using monoclonal antibodies. However, the method also uses electrophoresis, which is the same drawback as the aforementioned method. In addition, this method has further problems that it requires skills for operation and uses expensive monoclonal antibodies. Furthermore, these methods do not achieve measurement of protease in each individual cell, but they only measure total protease in the whole tissue. Therefore, they also have a problem in failing to provide information about infiltrative and metastatic activity of individual cancer cells.

Recently, a method is proposed wherein protease activity in vascular tissues is measured based on zymography (The FASEB Journal, Vol. 9, July, pp.974-980, 1995). According to the method, casein or gelatin modified with a fluorescent compound is used as a substrate for protease, and the method comprises the steps of forming a thin membrane of agarose containing the substrate on a microscope slide, placing unfixed tissue slice (6-10 *µ*m) on the surface of the thin membrane and incubating the slide at 37°C, and observing the digestion of the substrate by means of a fluorescence microscope (see, the protocol set out on page 975, right column, lines 6-18). Although this method has an advantage of direct measurement of protease in tissues, it requires agarose as an essential component to fix the substrate for protease on a microscope slide, which causes fluctuation in substrate digestion by protease and results in a problem of low reproducibility.

EP 0 319 334 discloses a method for assaying collagenolytic activitiy of mammalian biologic fluids comprising
obtaining a specimen of said tissue fluid;
applying said specimen to a slide having a collagen substrate film bonded to the surface thereof;
incubating said slide;
washing said slide;
staining said slide with a solution of a dye capable of staining collagen; and
determining the extend of dissolution of said collagen substrate from the extent of the unstained areas of the slide.

The incubation of the slide is achieved by baking the slides to cross-link the collagen and bond it to the slide surface.

US 4 137 082 teaches a photographic light sensitive material having at least one hydrophilic colloid layer containing (a) gelatin and/or a gelatin derivative and (b) at least one compound containing two or three vinylsulfonyl-moieties. The latter compounds can be used as gelatin hardeners in silver halide photographic light sensitive materials.

SU 1 178 761 teaches a method of identifying proteinase inhibitors by fractionation of protein in gels, characterized in that a sheet of filter paper, wetted with a solution of a proteinase in a buffer, is applied to the separating gel after fractionation, the paper is removed after fractionating, a photographic film is applied to the gel and incubated until bands of undegraded gelatin are detected on the photographic film, the photographic film is removed from the gel, a sheet of moistened filter paper is applied to it, and inhibitors are identified from the zones of undegraded gelatin in the form of dark bands on a transparent background on the photographic film or light bands on a dark background of the filter paper.

### Disclosure of the Invention

An object of the present invention is to provide a convenient and accurate method for measuring protease. More specifically, the object of the present invention is to provide a method for measuring protease which can achieve prompt and accurate determination of malignancy of cancer cells such as infiltrative and metastatic activity, pathological conditions of periodontal diseases and the like, degree of progress of destructive pathological conditions such as in rheumatism, and is useful for accurate diagnosis of, for example, prognosis of cancer, degree of progress of destructive pathological conditions.

Another object of the present invention is to provide a method for measuring protease which has the characteristic features mentioned above, and is capable of accurately measuring protease derived from cancer cells or other localized in test tissues.

A still further object of the present invention is to provide a thin membrane used for the method for measuring protease mentioned above.

The inventors of the present invention conducted various studies to achieve the foregoing objects. As a result, they found that, when a slice of tissue such as cancer tissue is brought into contact with a surface of a thin membrane containing a protease substrate such as gelatin together with a hardening agent, or when exudate collected from a morbid tissue such as a tissue of a periodontal disease is dropped onto the thin membrane, proteases contained in the sample digest the thin membrane and form the traces of digestion on the surface of the thin membrane. They also found that the method had much higher reproducibility compared to the method utilizing a thin membrane containing agarose (The FASEB Journal, Vol.9, July, pp.974-980, 1995), and achieved accurate measurement of protease activity in the samples. Furthermore, it was also found that proteases expressed in individual cells can be determined by preparing histopathological preparations and the above thin membrane preparations each by using one of continuous tissue slices and comparing the resulting preparations. The present invention was achieved on the basis of these findings.

The present invention thus provides a method for measuring protease which comprises the steps of (1) bringing a sample containing protease into contact with a thin membrane which comprises a protease substrate together with a hardening agent and is formed on a surface of a support; and wherein the protease substrate and the hardening agent are as specified in appended claim 1; and (2) detecting traces of digestion on the thin membrane formed by the action of the protease.
Further aspects and embodiments of the present invention are specified in the appended claims 2-10.

These methods are characterized in that a sample containing protease or a biological sample is brought into contact with a surface of a thin membrane and not with a support.

### Best Mode for Carrying Out the Invention

The methods of measuring protease according to the aforementioned embodiments basically comprise a step of bringing a sample containing protease into contact with a thin membrane (first step), and a step of detecting the trace of digestion on the thin membrane formed by the action of the protease (second step). The thin membranes for measuring protease used in the methods are formed on a surface of a support as monolayer or multilayer, and characterized in that they contain a protease substrate and a hardening agent as essential components. Therefore, the process according to the methods of the present invention is characterized in that the sample is not come into contact with the support at the beginning of the step of contact of the sample with the thin membrane. Furthermore, since the aforementioned thin membranes contain a hardening agent and are free from a binder such as agarose, the membranes contain a high density of a protease substrate such as gelatin to be digested by protease, and the breaking of the trace of digestion is prevented by the cross-linking of the protease substrate such as gelatin. For these reasons, the methods have characteristic features of excellent sensitivity and reproducibility of protease measurement.

The term "method of measurement" herein used should be construed in its widest sense including qualitative and quantitative analysis. In the methods of the present invention, the protease substrate is digested by proteases contained in a sample, and traces of digestion are formed on the thin membrane. These traces of digestion can be detected, for example, under a microscope, optionally after a staining, to verify the presence of proteases in the sample.

Proteases as the object of the measurement according to the present invention include, for example, matrix metalloproteinase (MMP) and matrix serine protease (MSP). These enzymes are explained in detail in "Molecular Mechanism of Cancer Metastasis", Ed. By T. Tsuruo, pp.92-107, Medical View Co., Ltd., 1993. Examples of proteases particularly suitable for the methods of the present invention include, for example, matrix metalloproteinases such as interstitial collagenase (MMP-1), gelatinase A (MMP-2) and gelatinase B (MMP-9); matrix serine proteases such as plasminogen activator (PA) and the like. However, the objects of the methods of the present invention are not limited to these proteases.
The protease substrates are selected from
collagen, gelatin, proteoglycan, fibronectin, laminin, elastin, and casein.

Preferably, collagen, gelatin, fibronectin, elastin or casein may be used, and gelatin, fibronectin and casein are more preferably used. The aforementioned substances may be used alone as the protease substrate, or in combination of two or more of them.

By using two or more different protease substrates in combination, a type of a protease contained in a biological sample can sometimes be accurately identified. For example, a method may be employed wherein one of two or more substantially continuous slices of a biological sample are brought into contact with a thin membrane containing a protease substrate; and the remaining slice is brought into contact with a thin membrane containing a protease substrate different from the protease substrate contained in the aforementioned thin membrane; and then traces of digestion formed on each of the thin membranes are compared. According to the method, the measurement can be performed by using three or more thin membranes each containing a different protease substrate. Furthermore, for example, a method may also be employed wherein a single thin membrane is prepared which comprises a first layer containing a protease substrate and a second layer containing a protease substrate different from the protease substrate contained in the first layer; a sample containing protease is brought into contact with the thin membrane; and then traces of digestion formed on each of the layers are compared. According to the aforementioned method, it is also possible to use a thin membrane comprising laminated three or more layers each containing a different protease substrate.

By using a protease inhibitor, identification of a protease that relates to the inhibitor or determination of properties of a protease may be facilitated. Examples of the protease inhibitors include, for example, tissue inhibitor of metaproteinase 1 (TIMP1), tissue inhibitor of metaproteinase 2 (TIMP2), large inhibitor of metalloproteinase (LIMP), chicken inhibitor of metalloproteinase (ChIMP), opostatin, platelet factor IV (PF-4), α₂ macroglobulin, EDTA, 1,10-phenanthroline, BB94, minocycline, matristatin, SC-44463, dithiothreitol (DTT) and the like. For example, a method may be employed wherein one of two or more substantially continuous slices of a biological sample is brought into contact with a thin membrane containing a protease substrate; and the remaining slices are brought into contact with other thin membranes containing a protease substrate together with a protease inhibitor; and then traces of digestion formed on each of the thin membranes are compared. In addiion, for example, a method may also be employed wherein a single thin membrane is prepared which contains a first layer containing a protease substrate and a second layer containing a protease substrate together with a protease inhibitor; a sample containing protease is brought into contact with the thin membrane; and then traces of digestion formed on each of the layers are compared. The methods utilizing protease inhibitors may further be combined with the aforementioned method utilizing two or more different protease substrates.

The hardening agent used for the manufacture of the thin membrane of the present invention has actions of promoting the curing of the thin membrane during the manufacture of the thin membrane containing the protease substrate and/or preventing the swell of the thin membrane after the formation of the thin membrane.

The hardening agent must have the above actions and must not substantially inhibit the reaction of protease and the protease substrate, and inorganic or organic hardening agents may be used. The hardening agent is selected from chromium salts (chrome alum, chromium acetate and other); aldehydes (formaldehyde, *glyoxal*, glutaraldehyde and other); N-methylol compounds (dimethylolurea, methyloldimethylhydantoin and other); dioxane derivatives (2,3-dibydroxydioxane and other); compounds, exhibiting activities through the activation of carboxyl group (carbenium, 2-naphthalenesulfonate, 1,1-bispyrrolidino-1-chloro-, pyridinium, 1-morpholinocarbonyl-3-(sulfonatoaminomethyl)-and other); activated vinyl compounds (1,3-bisvinylsulfonyl-2-propanol, 1,2-bis(vinylsulfonylacetamido)-ethane, bis(vinylsulfonylmethyl) ether, vinyl polymers having vinylsulfonyl groups in side chains, 1,3,5-triacryloyl-hexahydro-s-triazine, bis(vinylsulfonyl)methane and other); activated halogen compounds (2,4-dichloro-6-hydroxy-s-triazine, sodium salt thereof and other); mucohalogen acids (mucochloric acid, mucophenoxychloric acid and other); isoxazole compounds; dialdehyde starch; 2-chloro-6-hydroxytriazinylated gelatin, which may be used alone or in combination of two or more of them. Among them, vinylsulfonic acid-type hardening agents are preferred: An amount of the hardening agent is not particularly limited. For example, when gelatin is used as the protease substrate, about 0.1 to 20 mmol, more preferably about 0.3 to 10 mmol per 100 g of gelatin may be formulated from a viewpoint of detection performance.

As samples used for the method of the present invention, for example, biological samples isolated or collected from mammals including human may be used. The biological samples may be tissues or tissue exudate and the like. For example, cancer tissues from solid tumor tissues of lung cancer, stomach cancer, esophageal cancer, breast cancer, brain tumor and the like isolated or collected by surgical operation or histological examination; synovial fluid and bone tissue of rheumatoid arthritis; destructive morbid tissues such as periodontal ligament or bone tissue and exudate of alveolar pyorrhea; gingival crevicular exudate of periodontal diseases and the like may be used.

When a tissue is used as a sample, for example, a slice having a thickness of 1-10 *µ*m, preferably 5 µm may be prepared from a sample rapidly frozen in liquid nitrogen by using an apparatus for preparing frozen sections, and then the slice may be applied to a thin membrane to bring the sample contact with the thin membrane. When synovial fluid collected from a patient of rheumatoid arthritis is used as a sample, about 5-50 *µ*l, preferably about 20 *µ*l of synovial fluid can be dropped onto the thin membrane. When gingival crevicular fluid of periodontal disease is used as a sample, a filter paper piece may be inserted into gingival crevice to collect about 5-10 µl of gingival crevicular fluid, and the filter paper may be applied to a thin membrane. After the collection of gingival crevicular fluid, the gingival crevicular fluid may be optionally extracted from the filter paper using distilled water or a suitable buffer (for example, 50 mM Tris-HCl, pH 7.5, 10 mM CaCl₂, 0.2 M NaCl), and the extract may be dropped onto a thin membrane.

The thin membrane is preferably formed on a transparent or translucent support so that the presence of protease in a tissue can be observed under a microscope. Examples of such transparent or translucent support include, for example, glass and transparent or translucent plastic films made of polyethylene terephthalate, polycarbonate, polyimide, nylon, cellulose, triacetate and the like. As glass, it is preferred to use a microscope slide, and as a plastic film, it is preferred to use a polyethylene terephthalate film. However, the support is not limited to these examples, and any materials may be used so far that they can provide uniform thin membranes which are suitable for microscopic observation.

When a sample solution such as gingival crevicular fluid is dropped onto a thin membrane surface, traces of digestion may be detected along the peripheral circle of the circular applied area that is formed by the drop. In that case, detection under a microscope is not necessarily required, and accordingly, opaque supports can be used in addition to the above-explained supports. For example, paper, plastic paper, paper laminated with synthetic resin (for example, polyethylene, polypropylene, polystyrene, polyethylene naphthalate and other), metal plates (for example, plates of aluminium, aluminium alloy, zinc, iron, copper and other), paper or plastic films laminated or deposited by vaporization with the aforementioned metals and the like can be used. In the aforementioned embodiment, the support may be colored.

The thickness of the support is not particularly limited. As to glass supports, those having an usual thickness for a microscope slide (for example, approximately 2 to 3 mm) may be preferred. As to polyethylene terephthalate films, those having a thickness of about 100-250 µm, more preferably about 150-200 µm, and most preferably about 175 µm may be used. The thin membrane on the support can be formed as monolayer or multilayer, and the thin membrane should be prepared so as to have a surface as uniform as possible. For example, the thin membrane may preferably be formed so as to have a thickness of about 1-10 µm, more preferably about 4-6 µ m after drying.

For the preparation of the thin membrane, for example, a protease substrate, which is dispersed in water or an organic solvent such as methylene chloride, acetone, methanol and ethanol or a mixed solvent thereof, may be sprayed on a surface of a support and dried. As method for application, dip coasting method, roller coating method, curtain coating method, extrusion coating method and the like can be employed. However, methods for preparing the thin membrane are not limited to these examples, and methods conventionally used for the preparation of thin membranes in the field of photographic films, for example, may be appropriately employed. When gelatin is used as the protease substrate, a type of gelatin is not particularly limited. For example, alkali extracted bovine bone gelatin, alkali extracted swine cutis gelatin, acid extracted bovine bone gelatin, phthalation-treated bovine bone gelatin, acid extracted swine cutis gelatin and the like can be used.

When a thin membrane is formed on a support, an undercoat layer may be provided between the thin membrane and the support in order to improve the adhesion of the thin membrane and the support. For example, the undercoat layer may be formed by using a polymer or a copolymer obtained by the polymerization of one or more monomers selected from vinyl chloride, vinylidene chloride, butadiene, methacrylic acid, acrylic acid, itaconic acid, maleic anhydride and the like, or a polymer such as polyethyleneimine, epoxy resin, grafted gelatin, nitrocellulose and the like. When a polyester support is used, adhesion between the support and the thin membrane may sometimes be improved by subjecting the surface of the support to corona discharge treatment, ultraviolet irradiation or glow discharge treatment instead of providing the undercoat layer.

The wording "a thin membrane formed on a surface of a support" and equivalents thereof should not be construed to exclude those with one or more undercoat layers and/or a surface treatment of the support. Means to improve the adhesion of the thin membrane and the support are not limited to those mentioned above, and for example, those conventionally used in the field of photographic film and other can be appropriately used.

For the preparation of the thin membrane, other components such as dyes, pigments, antiseptics, stabilizers and the like may optionally be added in addition to the components mentioned above. These components may be appropriately chosen and employed so long as they do not substantially inhibit the reaction of protease and the protease substrate. For example, as the dyes, those disclosed in the Japanese Patent Unexamined Publication (KOKAI) No. (Hei)6-102624/1994 (dyes specifically represented by formula 1-1 on page 9 to formula 63 on page 47) can be used. As methods for adding dyes, those disclosed in the Japanese Patent Unexamined Publication (KOKAI) No. (Hei)5-313307/1993 (methods specifically explained from paragraph [0037] on page 11 to paragraph [0044] on page 12) can be used. The addition of a dye or a pigment may facilitate the detection of traces of digestion. For this purpose, a metal and a metal oxide may also be formulated other than the dye and the pigment. When a substance such as metals, metal oxides, dyes, and pigments is added, the thin membrane as the final product may preferably have a maximum transmission density of 0.01 or higher at a wavelength ranging from 400 nm to 700 nm. One or more of these substances may be formulated in the thin membrane, and different substances such as dyes in different colors may be added to each of the layers constituting the thin membrane. If agarose is used for the manufacture of the thin membrane, the reproducibility of protease detection may be lowered. Therefore, it is not preferred to use agarose for the manufacture of the thin membrane of the present invention.

According to the methods of the present invention, a sample containing protease is brought into contact with a thin membrane by, for example, applying a tissue slice to the thin membrane or dropping a liquid sample onto the thin membrane, and then incubation is carried out preferably in a humidified box at 37°C for a period of, for example, about 1-24 hours, preferably about 2-12 hours, more preferably about 3-6 hours for tissue slices, or about 0.5-12 hours, preferably about 1-6 hours, more preferably about 1-3 hours for liquid samples. When proteases are contained in the sample, the protease substrate in the thin membrane is degraded by the proteases, and the traces of digestion are formed on the thin membrane. The thin membrane is subjected to observation with the naked eyes or under a microscope, optionally after staining, to verify the presence of the protease. The evaluation may be performed spectrometrically by means of a spectrophotometer.

When gelatin is used as the protease substrate, it is preferred to observe traces of digestion after a gelatin staining. The gelatin staining can be performed according to a conventional method by using, for example, 1% Amido Black or Coomassie Blue. For example, a gelatin thin membrane is stained black dark-blue by the gelatin staining using Amido Black. When traces of gelatin digestion are formed on the surface of the gelatin thin membrane by protease, white spots not stained appear because gelatin is absent in the portions of the traces of digestion. A gelatin thin membrane is formed on a photographic film, and accordingly, a photographic film (for example, Neopan F, Fuji Photo Film Co., Ltd.) may be used as the thin membrane after light exposure and conventional development, fixation, rinsing, and drying treatment. In this case, traces of digestion can be observed as white portions on a black light-exposed film.

According to another embodiment of the method of the present invention, the presence of protease derived from each individual cells in a tissue can be accurately determined by preparing a continuous frozen sections from a cancer tissue and other, preparing a conventional histopathological preparation of one of two substantially continuous slices such as a hematoxylin/eosin staining slice, treating the other slice according to the method of measurement of the present invention, and comparing the results of each observation. This embodiment of method for measuring protease enables accurate determination of malignancy of individual cancer cells (infiltrative and metastatic activity) present in tissues, and definite diagnosis of the prognosis of the cancer. Furthermore, by quantitative measurement of protease in a sample of synovial fluid from rheumatoid arthritis, gingival crevicular fluid and the like, pathological conditions and the degree of progress of diseases can be accurately determined. For the quantitative measurement of proteases in a sample, it is preferred to prepare a calibration curve using a standard solutions prepared beforehand.

### Examples

The present invention will be explained more specifically by referring to the following examples. However, the scope of the present invention is not limited to these examples.

### Example 1: Preparation of thin membrane for the measurement of protease

Alkali extracted bovine bone gelatin (10 g) was dissolved in pure water (127 g), and the solution was added with 1,2-bis(vinylsulfonylacetamido)ethane (2%, 0.8 ml) as a hardening agent. The solution was uniformly applied to a polyethylene terephthalate film provided with an undercoat layer so as to obtain a dried membrane having the thickness of about 5 µm, and then the membrane was dried to obtain a gelatin thin membrane. The gelatin thin membrane was stored at room temperature before use.

### Example 2: Preparation of thin membrane for the measurement of protease

Alkali extracted bovine bone gelatin solution was prepared in the same manner as in Example 1, and the solution was applied to a microscope slide so as to obtain a membrane having the thickness of about 6 µm after drying, and then the membrane was dried to obtain a gelatin thin membrane. Furthermore, by using each of alkali extracted swine cutis gelatin, acid extracted bovine bone gelatin, phthalation-treated bovine bone gelatin, acid extracted swine cutis gelatin (#G2625, Sigma) and acid extracted swine cutis gelatin (#G2500, Sigma) instead of the alkali extracted bovine bone gelatin, gelatin thin membranes were prepared.

### Example 3: Measurement of proteases

As protease liquid samples, solutions each containing matrix metalloproteinase (MMP)-1, MMP-2 and MMP-9 (Yagai Co., Ltd.) at a concentration of from 2 pg/ml to 200 ng/ml were used. As biological samples, gingiva and gingival crevicular fluid (GCF) collected from periodontal disease patients and culture supernatants of pathogenic bacteria causing periodontal disease (P. Gingivalis #381 strain; A. actinomycetemcomitans Y4 strain; and P. intermedia ATCC 25611 strain) were used. About 10 *µ*l of liquid samples were dropped onto each of the gelatin thin membranes obtained in Example 2, and tissue samples prepared as frozen slices of about 5 µm were applied to each of the gelatin thin membranes. The gelatin thin membranes were placed in a humidified box and incubated at 37°C for 4-16 hours, and then stained with Coomassie Blue.

As a result, portions not stained due to the gelatin digestion by protease (portions of white spots: traces of gelatin digestion) were observed in all of the gelatin thin membranes. In particular, gelatin thin membranes of the phthalation-treated bovine bone gelatin gave conspicuous gelatin digestion for liquid samples, and gelatin thin membranes of acid extracted swine cutis gelatin also gave distinct traces of gelatin digestion for liquid samples and tissue slices. As to MMP-1, MMP-2 and MMP-9, protease activities were observed at a concentration of 200 ng/ml after 4 hours, at 20 ng/ml after 8 hours, and at a concentration ranging from 20 to 200 pg/ml after 16 hours. Most of the gingival crevicular fluids required 8-16 hours until protease activity was recognized, and it was suggested that the amount of protease was in a range of from 2 pg/ml to 20 ng/ml. Protease activities in tissue slices were recognized at sites of crevicular epithelium, circumference epithelium and subepithelial connective tissue where strong inflammatory cell infiltration was observed.

### Example 4: Measurement of protease in cancer tissues

As test tissue samples, surgical specimens of glossa epidermoid cancer, lung cancer and esophageal cancer were cut into pieces of about 0.5 cm thickness × 2 cm width, rapidly frozen in liquid nitrogen, and then stored at -80°C. Continuous slices having a thickness of 5 *µ*m were prepared from these samples using a frozen section preparing apparatus, and one of the slices was affixed to a microscope slide and dried. Then, the slice was fixed with 10% formalin for 5 minutes, and stained with hematoxylin/eosin in a conventional manner. The other continuous slices were affixed on the gelatin thin membranes produced in Example 2 and Example 3, and the membranes were placed in a humidified box and incubate at 37°C for 3-6 hours.

After the incubation, the gelatin thin membranes were stained with 1% Amido Black solution, dried, and microscopically examined. Portions where protease activities were expressed gave traces of gelatin digestion as white spots, whereas the other portions were in dark blue black. For all of the cancer tissues, the traces of gelatin digestion were recognized in individual cancer cells forming cancer alveolus lesions, and the cells located in peripheries of cancer alveolus lesions gave particularly distinct traces of gelatin digestion. Weak gelatin digestion was also observed in normal platycytes, and correlating to the progress of atypical epithelium growth, stronger gelatin digestion was recognized.

### Example 5: Clinical case (buccal cavity maxillary gingiva cancer)

Cancer cells in a sample were those of low-differentiated epidermoid cancer which formed an alveolus lesion structure, and they destroyed bone tissues and showed strong infiltration. Portions of gelatin thin membrane, corresponding to the cancer cells in the cancer alveolus lesions, were digested to give traces of gelatin digestion as white spots on the gelatin thin membrane preparation, and portions of the gelatin thin membrane, corresponding to the cells located in peripheries of the cancer alveolus lesions, particularly showed distinct traces of gelatin digestion. At portions corresponding to focal inflammatory cell infiltration sites, remarkable granular traces of gelatin digestion were formed. When the cancer alveolus lesions were observed in magnification, distinct traces of gelatin digestion were recognized in the cells of growth sites located at peripheries of the cancer alveolus lesions, and granular traces of gelatin digestion were observed also at fibroblasts in interstitium adjacent to the cancer alveolus lesions.

### Example 6: Clinical case (lingual cancer)

Cancer cells in a sample were those of undifferentiated epidermoid cancer, and they formed cancer alveolus lesions in various sizes. Every portions corresponding to the cells located in peripheries of the cancer alveolus lesions showed distinct traces of gelatin digestion, and the traces of gelatin digestion at sites corresponding to the cells of growth regions were particularly notable. Granular traces of gelatin digestion were observed also at portions corresponding to focal inflammatory cell infiltration sites.

### Example 7: Clinical case (severe epithelium dysplasia of oral mucosa)

In a hematoxylin/eosin stained sample, severe epithelium dysplasia was recognized in epithelium, and hyperplasia of prickle cells and multiple stratification of basal cells were observed. In particular, polymorphism and atypical cell growth were observed in basal cells. On the gelatin thin membrane, distinct traces of gelatin digestion were recognized at portions corresponding to the hyperplastic prickle cell layer and the granulocyte layer, whereas only punctate traces of gelatin digestion were recognized in the basal cell layer. These results suggest that turnover of epithelial cells was very rapid, whereas basal cells infiltrated into the epithelial connective tissues.

When the hyperplasia of prickle cells and the multiple stratification of basal cells were observed in magnification, traces of gelatin digestion were recognized on portions corresponding to both of the cell layers, and in particular, gelatin was markedly digested at portions corresponding to the prickle cells and stratified basal cells. On the other hand, gelatin was less digested by basal cells in monolayer or bilayer compared to stratified basal cells. The stratified basal cells were deformed into a spindle shape, and polymorphism and heteromorphism of the cells were observed. Portions corresponding to the cells exhibiting heteromorphism and growing tendency formed distinct traces of gelatin digestion on the gelatin thin membrane.

### Example 8: Measurement of protease activity in a liquid sample

About 20 µl of synovial liquid of rheumatism patients was dropped on the gelatin thin membrane, and the membrane was incubated in a humidified box at 37°C for 1-3 hours. The gelatin thin membrane was then stained with 1% Amido Black. Distinct traces of gelatin digestion were observed at a peripheral circle of the applied circular portion on the gelatin thin membrane formed by the dropping. In particular, when a thin membrane of phthalation-treated bovine bone gelatin was used, particularly conspicuous traces of gelatin digestion were observed

### Example 9: Measurement of protease activity in periodontal diseases

Saliva and dental plaque on tooth surface were removed with pledgets as completely as possible for simple exclusion of moisture, and then a piece of Perio paper was inserted in gingival crevice and left stand for 90 seconds to allow the absorption of gingival crevicular fluid (about 5-10 µl) in the Perio paper. The piece of Perio paper was extracted with 150 *µ*l of a buffer (50 mM Tris-HCl, pH 7.5, 10 mM CaCl₂, 0.2 M NaCl), and protease was measured according to the same method as Example 3 using the extract as a sample solution. As a result, distinct traces of gelatin digestion were observed along the peripheral circle of the applied circular portion on the gelatin thin membrane formed by the dropping of the gingival crevicular fluid.

### Example 10: Preparation of the thin membrane for the measurement of protease

Alkali extracted bovine bone gelatin (15 g) was dissolved in pure water (123 g), and the solution was added with 1,2-bis(vinylsulfonylacetamido)ethane (4% aqueous solution, 0.6 ml) as a hardening agent. The solution was uniformly applied to an object glass by a wire bar coater so as to obtain a dried membrane having the thickness of about 7 *µ*m, and then the membrane was dried to obtain a gelatin thin membrane (single layer thin membrane: Sample 101). The thin membrane was stored at room temperature before use. By using the protease substrates shown in Table 1 instead of the alkali extracted bovine bone gelatin, and changing or supplementing the hardening agent, the additive, and the support as shown in Table 1, Samples 102-130 were produced in the same manner as Sample 101. Upon application, an application aid was used as required. In the table, Dye 1 represents Amido Black, and Pigment 1 represents copper phthalocyanine.

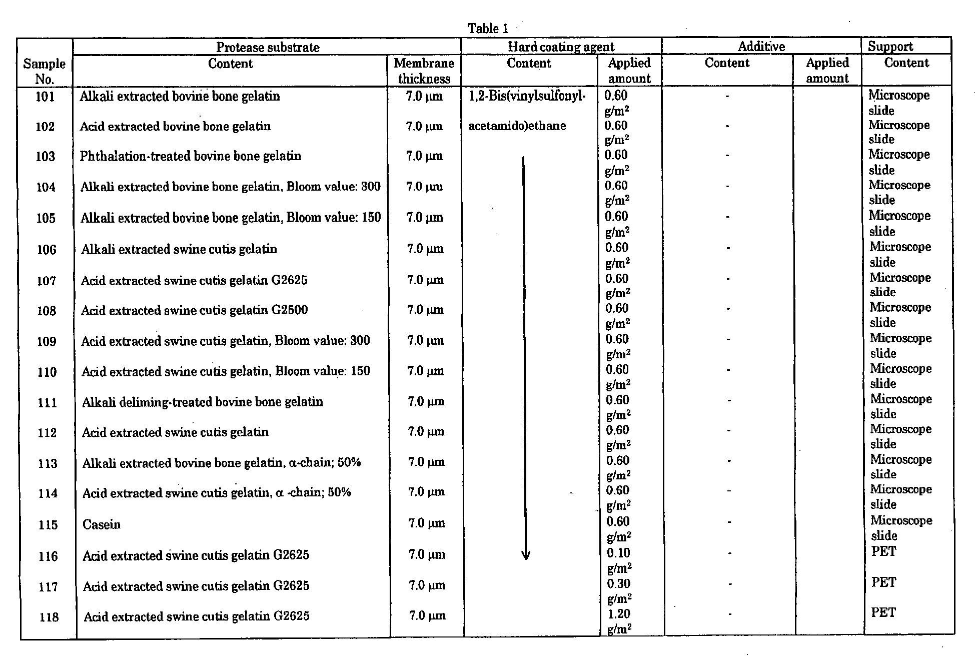

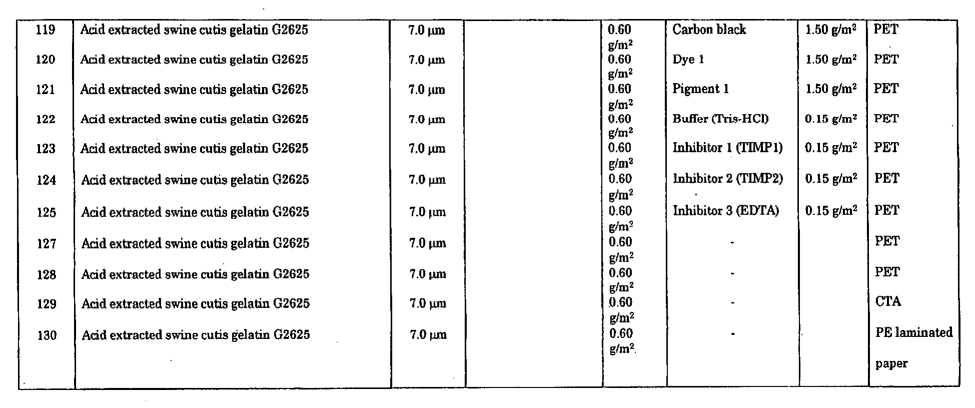

### Example 11: Preparation of thin membrane for protease assay

A collagen I solution (Wako Pure Chemical Industries, 3 mg/ml) was uniformly applied to a microscope slide by a wire bar coater so as to obtain a dried membrane having the thickness of about 1 *µ*m, and then the membrane was dried to obtain a gelatin thin membrane (single layer thin membrane: Sample 131). The thin membrane was stored at room temperature before use. By using the protease substrates shown in Table 2 instead of collagen I, and changing or supplementing the hardening agent, the additive, and the support as shown in Table 2, Samples 132-160 were produced in the same manner as Sample 131. Upon application, an application aid was used as required.

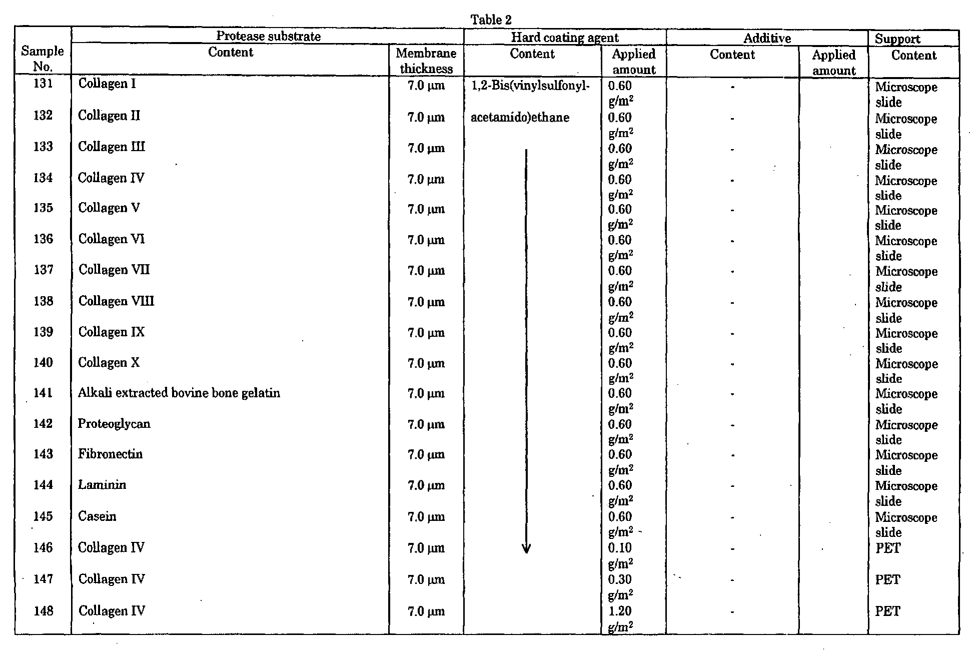

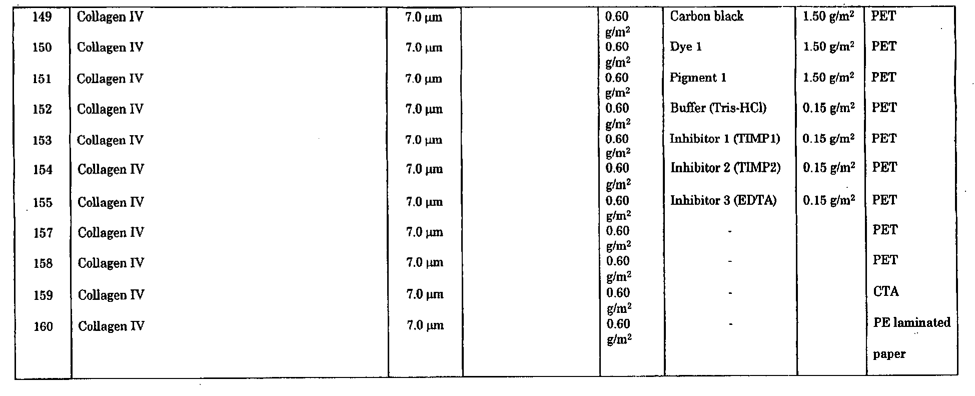

Samples 161-170 were prepared in the same manner as Samples 121-130, except that a slide coater was used instead of the wire bar coater for application. As drying conditions, a process is applied wherein the samples was optionally once cooled to 10°C as required, and dried at ordinary temperature and humidity.

### Example 12: Protease activity assay

About 10 *µ*l of the MMP-2 described in Example 3 was used as a protease liquid sample, and the solution was dropped onto each of the thin membranes obtained in Example 11. The thin membranes were placed in a humidified box, and incubated at 37°C for 4-16 hours. Then, transparent samples were stained with an Amido Black solution. As the evaluation of the activity, (1) observation with the naked eyes, (2) the density measurement of microportions by microdensitometry, and (3) the measurement of a membrane thickness using a contact membrane thickness measuring apparatus were performed for each of the samples. The results are shown in Table 3.

**Table 3**

| Sample No. | Traces of digestion | Optical density | Membrane thickness *(*µm) |
|---|---|---|---|
| 101 | Present | 0.08 | 0.4 |
| 102 | Present | 0.08 | 0.4 |
| 103 | Present | 0.04 | 0.2 |
| 104 | Present | 0.08 | 0.4 |
| 105 | Present | 0.02 | 0.0 |
| 106 | Present | 0.04 | 0.4 |
| 107 | Present | 0.02 | 0.0 |
| 108 | Present | 0.02 | 0.0 |
| 109 | Present | 0.04 | 0.4 |
| 110 | Present | 0.02 | 0.0 |
| 111 | Present | 0.04 | 0.4 |
| 112 | Present | 0.04 | 0.4 |
| 113 | Present | 0.02 | 0.2 |
| 114 | Present | 0.02 | 0.2 |
| 115 | Present | 0.08 | 0.4 |
| 116 | Present | 0.02 | 0.0 |
| 117 | Present | 0.02 | 0.0 |
| 118 | Present | 0.10 | 0.5 |
| 119 | Present | 0.02 | 0.0 |
| 120 | Present | 0.02 | 0.0 |
| 121 | Present | 0.02 | 0.0 |
| 122 | Present | 0.02 | 0.0 |
| 123 | Absent | 4.50 | 7.0 |
| 124 | Absent | 4.50 | 7.0 |
| 125 | Absent | 4.50 | 7.0 |
| 127 | Present | 0.02 | 0.0 |
| 128 | Present | 0.02 | 0.0 |
| 129 | Present | 0.02 | 0.0 |
| 130 | Present | 0.02 | 0.0 |
| 131 | Present | 0.08 | 0.4 |
| 132 | Present | 0.08 | 0.4 |
| 133 | Present | 0.04 | 0.2 |
| 134 | Present | 0.08 | 0.4 |
| 135 | Present | 0.02 | 0.0 |
| 136 | Present | 0.04 | 0.4 |
| 137 | Present | 0.02 | 0.0 |
| 138 | Present | 0.02 | 0.0 |
| 139 | Present | 0.04 | 0.4 |
| 140 | Present | 0.02 | 0.0 |
| 141 | Present | 0.04 | 0.4 |
| 142 | Present | 0.04 | 0.4 |
| 143 | Present | 0.02 | 0.2 |
| 144 | Present | 0.02 | 0.2 |
| 145 | Present | 0.08 | 0.4 |
| 146 | Present | 0.02 | 0.0 |
| 147 | Present | 0.02 | 0.0 |
| 148 | Present | 0.10 | 0.5 |
| 149 | Present | 0.02 | 0.0 |
| 150 | Present | 0.02 | 0.0 |
| 151 | Present | 0.02 | 0.0 |
| 152 | Present | 0.02 | 0.0 |
| 153 | Absent | 4.50 | 7.0 |
| 154 | Absent | 4.50 | 7.0 |
| 155 | Absent | 4.50 | 7.0 |
| 157 | Present | 0.02 | 0.0 |
| 158 | Present | 0.02 | 0.0 |
| 159 | Present | 0.02 | 0.0 |
| 160 | Present | 0.02 | 0.0 |
| Control (untreated) | Absent | 4.50 | 7.0 |

In all of the thin membranes free from the protease inhibitors, white spots where the protease substrate was digested by the protease (traces of digestion) were observed, and optical density and membrane thickness of these portions were reduced compared to the surrounding portions. On the other hand, in the samples added with the protease inhibitors, traces of digestion were not observed because the protease activity was inhibited. Samples 160-170 prepared by using a slide coater gave the same results as the samples prepared by a bar coater.

### Example 13: Measurement of protease activity

Sample 101 obtained in Example 11 was subjected to the measurement to obtain a calibration curve according to the same method as Example 12 by using solutions containing matrix metalloproteinase (MMP)-1 at a concentration of from 2 pg/ml to 200 ng/ml as protease liquid samples. The results are shown in Table 4.

**Table 4**

| Amount of MMP added (g/ml) | Traces of digestion | Optical density | Membrane thickness (µm) |
|---|---|---|---|
| 10⁻⁷ | Present | 0.2 | 0.1 |
| 10⁻⁸ | Present | 0.2 | 0.1 |
| 10⁻⁹ | Present | 0.2 | 0.1 |
| 10⁻¹⁰ | Present | 1.0 | 0.5 |
| 10⁻¹¹ | Slightly present | 4.0 | 6.0 |
| 10⁻¹² | Absent | 4.5 | 7.0 |
| No addition | Absent | 4.5 | 7.0 |

The results of Table 4 were plotted in a graph to obtain the concentration of metalloproteinase (MMP)-1 that gave the optical density of the traces of digestion (2.6) when biological samples were used. As a result, it was found that the concentration of the protease in the biological samples was about 40 pg/ml.

### Example 14: Protease activity assay

Biological samples of gingiva, gingival crevicular fluid, and periodontal tissue samples collected from periodontal disease patients were affixed onto Samples 101-160 of the thin membranes as about 5 µm frozen slices. The thin membranes were placed in a humidified box, and incubated at 37°C for 4-16 hours. Then, transparent samples were stained with an Amido Black solution. For the portions of the samples where the activity was clearly observed, evaluation of the activity was performed by (1) observation with the naked eyes, (2) the density measurement of microportions by microdensitometry, and (3) the measurement of a membrane thickness using a contact membrane thickness measuring apparatus. The results are shown in Table 5.

**Table 5**

| Sample No. | Traces of digestion | Optical density | Membrane thickness (*µ*m) |
|---|---|---|---|
| 101 | Present | 0.10 | 0.6 |
| 102 | Present | 0.10 | 0.6 |
| 103 | Present | 0.06 | 0.4 |
| 104 | Present | 0.10 | 0.6 |
| 105 | Present | 0.02 | 0.0 |
| 106 | Present | 0.04 | 0.4 |
| 107 | Present | 0.02 | 0.0 |
| 108 | Present | 0.02 | 0.0 |
| 109 | Present | 0.04 | 0.4 |
| 110 | Present | 0.02 | 0.0 |
| 111 | Present | 0.04 | 0.4 |
| 112 | Present | 0.04 | 0.4 |
| 113 | Present | 0.02 | 0.2 |
| 114 | Present | 0.02 | 0.2 |
| 115 | Present | 0.08 | 0.4 |
| 116 | Present | 0.02 | 0.0 |
| 117 | Present | 0.02 | 0.0 |
| 118 | Present | 0.16 | 0.8 |
| 119 | Present | 0.02 | 0.0 |
| 120 | Present | 0.02 | 0.0 |
| 121 | Present | 0.02 | 0.0 |
| 122 | Present | 0.02 | 0.0 |
| 123 | Absent | 4.50 | 7.0 |
| 124 | Absent | 4.50 | 7.0 |
| 125 | Absent | 4.50 | 7.0 |
| 127 | Present | 0.02 | 0.0 |
| 128 | Present | 0.02 | 0.0 |
| 129 | Present | 0.02 | 0.0 |
| 130 | Present | 0.02 | 0.0 |
| 131 | Present | 0.12 | 0.6 |
| 132 | Present | 0.08 | 0.5 |
| 133 | Present | 0.06 | 0.4 |
| 134 | Present | 0.12 | 0.6 |
| 135 | Present | 0.02 | 0.0 |
| 136 | Present | 0.04 | 0.4 |
| 137 | Present | 0.02 | 0.0 |
| 138 | Present | 0.02 | 0.0 |
| 139 | Present | 0.04 | 0.4 |
| 140 | Present | 0.02 | 0.0 |
| 141 | Present | 0.04 | 0.4 |
| 142 | Present | 0.04 | 0.4 |
| 143 | Present | 0.02 | 0.2 |
| 144 | Present | 0.02 | 0.2 |
| 145 | Present | 0.10 | 0.5 |
| 146 | present | 0.02 | 0.0 |
| 147 | Present | 0.02 | 0.0 |
| 148 | Present | 0.16 | 0.8 |
| 149 | Present | 0.02 | 0.0 |
| 150 | Present | 0.02 | 0.0 |
| 151 | Present | 0.02 | 0.0 |
| 152 | Present | 0.02 | 0.0 |
| 153 | Absent | 4.50 | 7.0 |
| 154 | Absent | 4.50 | 7.0 |
| 155 | Absent | 4.50 | 7.0 |
| 157 | Present | 0.02 | 0.0 |
| 158 | Present | 0.02 | 0.0 |
| 159 | Present | 0.02 | 0.0 |
| 160 | Present | 0.02 | 0.0 |
| Control (untreated) | Absent | 4.50 | 7.0 |

In the method wherein frozen slices of biological samples are directly placed, traces of digestion of the protease substrate by the protease (portions of white spaces) were also formed on each thin membrane, and optical density and membrane thickness of these portions were reduced compared to surrounding portions.

### Example 15: Preparation of thin membrane for protease assay

Alkali extracted bovine bone gelatin (15 g) was dissolved in pure water (123 g), and the solution was added with 1,2-bis(vinylsulfonylacetamido)ethane (4% aqueous solution, 0.6 ml) as a hardening agent. The solution was uniformly applied to a microscope slide by a wire bar coater so as to obtain a dried membrane having the thickness of about 7 µm, and then the membrane was dried to obtain a thin membrane. Alkali extracted bovine bone gelatin (15 g) was dissolved in pure water (123 g), and the solution was added with 1,2-bis(vinylsulfonylacetamido)ethane (4% aqueous solution, 0.6 ml) as a hardening agent and polymethyl methacrylate particles (average diameter; 2 µm). This solution was uniformly applied to the surface of the already prepared dried thin membrane by a wire bar coater so as to obtain a dried membrane having the thickness of about 7 *µ*m, and then the membrane was dried to obtain a thin membrane (multiple-layer thin membrane: Sample 301). The thin membranes were stored at room temperature before use. Those thin membranes shown in Table 6 were prepared by changing or supplementing the protease substrate and the hardening agent. For the application, application aids and viscosity modifiers were used as required. As Blue dye 1, Green dye 1 and Red dye 1 specified in the table, the following compounds were used, respectively.

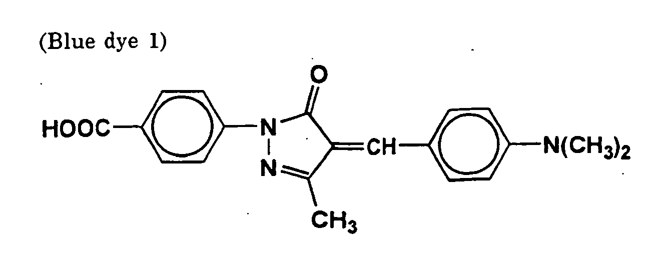

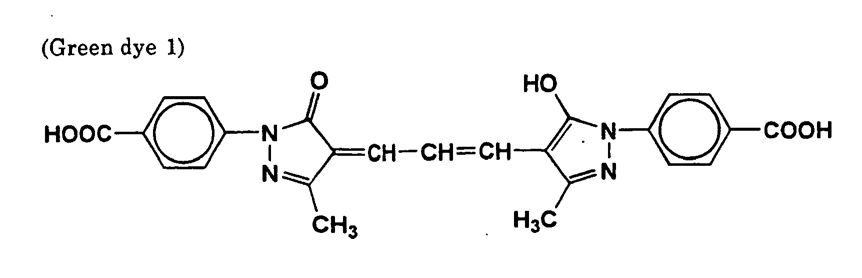

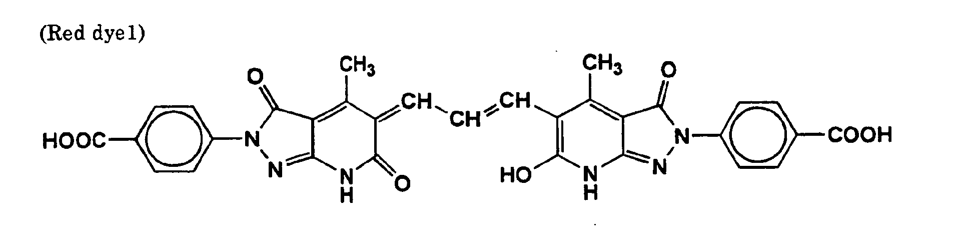

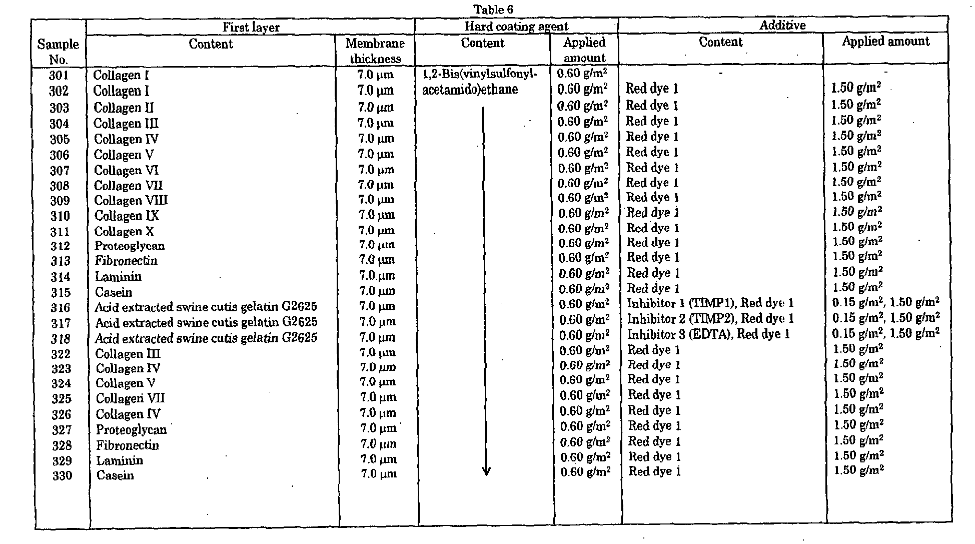

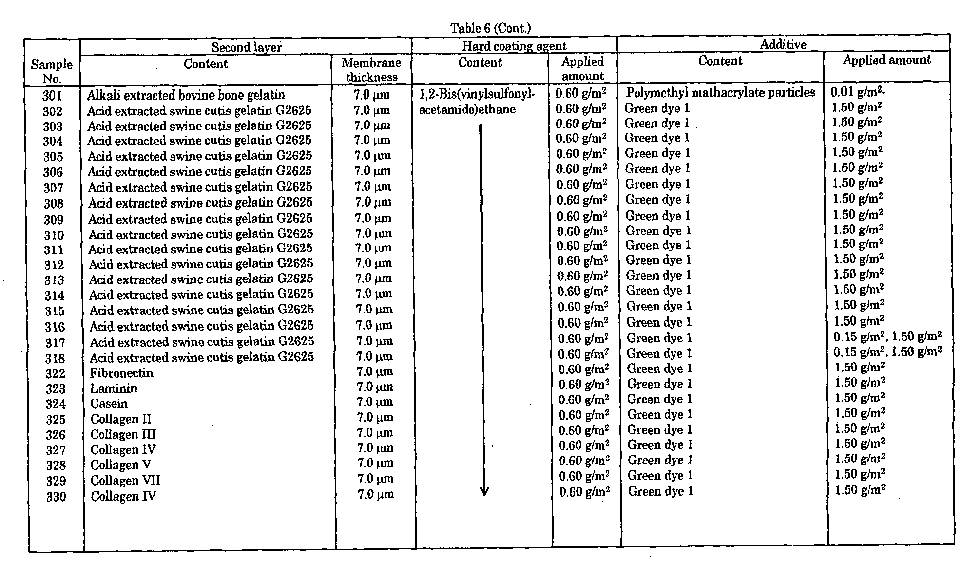

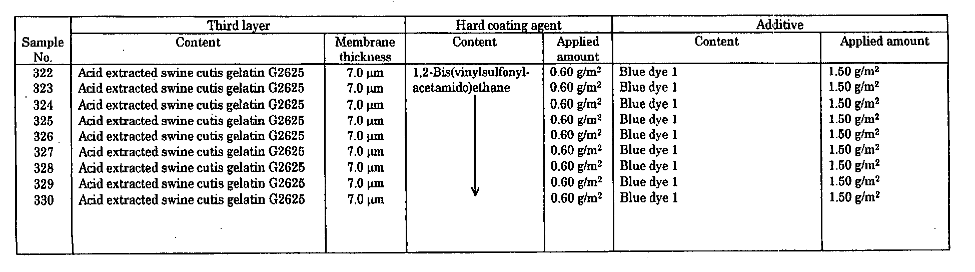

Samples 331-360 were prepared in the same manner as the preparation of Samples 301-330, except that a slide coater was used instead of the wire bar coater for application. As drying conditions, a process is applied wherein the samples was optionally once cooled to 10°C as required, and dried at ordinary temperature and humidity.

### Example 16: Protease activity assay

Protease activity assay was performed in the same manner as Example 12. The results are shown in Table 7. From these results, it was found that the protease activity was detectable with extreme distinctness by means of the multiple-layer thin membranes. Samples 331-360 prepared by using a slide coater gave the same results as the samples prepared by a bar coater.

**Table 7**

| Sample No. | Traces of digestion | | | Optical density | | | Membrane thickness | | |
|---|---|---|---|---|---|---|---|---|---|
| | First layer | Second layer | Third layer | First layer | Second layer | Third layer | First layer | Second layer | Third layer |
| 301 | Present | Present | | 0.12 | 0.02 | | 0.6 µm | 0.6 µm | |
| 302 | Present | Present | | 0.08 | 0.04 | | 2.0 µm | 0.5 µm | |
| 303 | Present | Present | | 0.06 | 0.02 | | 1.0 µm | 0.4 µm | |
| 304 | Present | Present | | 0.12 | 0.02 | | 3.0 µm | 0.6 µm | |
| 305 | Present | Present | | 0.02 | 0.04 | | 4.0 µm | 0.0 µm | |
| 306 | Present | Present | | 2.02 | 0.02 | | 2.0 µm | 0.4 µm | |
| 307 | Present | Present | | 3.50 | 0.04 | | 2.0 µm | 0.0 µm | |
| 308 | Present | Present | | 1.50 | 0.04 | | 1.0 µm | 0.0 µm | |
| 309 | Present | Present | | 3.50 | 0.02 | | 3.0 µm | 0.4 µm | |
| 310 | Present | Present | | 2.02 | 0.02 | | 2.0 µm | 0.0 µm | |
| 311 | Present | Present | | 3.50 | 0.04 | | 1.0 µm | 0.4 µm | |
| 312 | Present | Present | | 1.50 | 0.02 | | 3.0 µm | 0.4 µm | |
| 313 | Present | Present | | 3.50 | 0.02 | | 4.0 µm | 0.2 µm | |
| 314 | Present | Present | | 2.50 | 0.04 | | 2.0 µm | 0.2 µm | |
| 315 | Present | Present | | 0.10 | 0.02 | | 0.5 µm | 0.5 µm | |
| 316 | Absent | Present | | 4.50 | 0.04 | | 7.0 µm | 0.0 µm | |
| 317 | Absent | Present | | 4.50 | 0.04 | | 7.0 µm | 0.0 µm | |
| 318 | Absent | Present | | 4.50 | 0.02 | | 7.0 µm | 0.0 µm | |
| 322 | Present | Present | Present | 2.02 | 0.02 | 0.04 | 3.0 µm | 0.0 µm | 0.0 µm |
| 323 | Present | Present | Present | 3.50 | 2.02 | 0.02 | 3.0 µm | 2.0 µm | 0.4 µm |
| 324 | Present | Present | Present | 1.50 | 3.50 | 0.04 | 2.0 µm | 1.0 µm | 0.4 µm |
| 325 | Present | Present | Present | 3.50 | 1.50 | 0.02 | 1.0 µm | 3.0 µm | 2.0 µm |
| 326 | Present | Present | Present | 2.50 | 3.50 | 0.02 | 3.0 µm | 2.0 µm | 0.2 µm |
| 327 | Present | Present | Present | 2.04 | 2.50 | 0.04 | 4.0 µm | 1.0 µm | 0.5 µm |
| 328 | Present | Present | Present | 1.23 | 0.02 | 0.02 | 2.0 µm | 3.0 µm | 0.0 µm |
| 329 | Present | Present | Present | 3.12 | 0.02 | 0.04 | 4.0 µm | 4.0 µm | 0.0 µm |
| 330 | Present | Present | Present | 2.02 | 0.02 | 0.04 | 3.0 µm | 2.0 µm | 0.8 µm |
| Untreated, | Absent | Absent | Absent | 4.50 | 4.50 | 4.50 | 7.0 µm | 7.0 µm | 7.0 µm |
| Staining | | | | | | | | | |
| alone | | | | | | | | | |

### Example 17: Preparation of thin membrane for measuring protease and the measurement of protease activity

Alkali extracted bovine bone gelatin (15 g) was dissolved in pure water (123 g), and the solution was added with 1,2-bis(vinylsulfonylacetamido)ethane (4%, 0.6 ml) as a hardening agent. Samples 501-530 were prepared in the same manner as in the preparation of Samples 331-360, except that the above solution was applied as a back layer by using a slide coater, and dried to obtain thin membranes. Samples 531-560 were prepared by applying a polymer latex layer on the back layer of Sample 501-530, and drying the layer. the measurements of protease activity using these samples in the same manner as the aforementioned measurements using Samples 331-360 gave similar results. No curling was occurred in these samples, and the membranes were excellent in handling property.

### Example 18: Measurement of protease using a photographic film

A Neopan F (monochrome film for photography, Fuji Photo Film Co., Ltd.) was completely exposed to light, and then the film was developed, fixed, rinsed and dried in a conventional manner. The photographic film was used as a gelatin thin membrane for protease measurement, and the measurement of protease activity was performed in the same manner as Example 4 by affixing slices of the cancer tissues prepared in Example 4 on this film. As a result, portions where protease was absent were remained black, whereas traces of digestion similar to those observed on the stained thin membranes prepared in Example 4 were observed as white spots. However, detection sensitivity of the film was lower than the thin membranes produced in Examples 1, 2, 10, 11, 15 and 17.

### Example 19: Measurement of protease utilizing an emulsion for radiography and results of the measurement (Comparative example)

Biological samples of gingiva, gingival crevicular fluid, and periodontal tissue samples collected from periodontal disease patients were affixed onto a microscope slide as about 5 µm frozen slices. Emulsion for radiography NRM2 or NRH2 (KONICA CORPORATION) diluted with water was applied on the slices, and dried to form thin membranes. The thin membranes were placed in a humidified box, and incubated at 37°C for 4-16 hours. Then, the membranes were stained with Amido Black or subjected to a monochromic development. As a result, almost no protease activity was detected. Trace of activity was observed in samples incubated for 14 hours and subjected to monochromic development, however, indication of the activity area was very ambiguous, and their protease detection performance was far inferior to that of the thin membranes of the present invention.

### Industrial Applicability

The methods of the present invention are characterized in that they enable accurate and convenient measurement of protease derived from specific lesions localized in tissues or individual cells in tissues, and in addition, determination can be completed in a short period of time. Therefore, the methods of the present invention are useful for accurate diagnosis of malignancy of cancer cells such as infiltration and metastasis activities, degree of progress of periodontal disease such as periodontitis, destructive pathological conditions of rheumatism, alveolar pyorrhea and the like. According to the methods of the present invention, protease activity can be measured from extremely small amount of samples, and thin membranes after the measurement may be stored permanently as fixed preparations.

## Claims

1. A method for measuring protease which comprises the steps of:
(1) bringing a sample containing protease into contact with a thin membrane which comprises a protease substrate together with a hardening agent and is formed on a surface of a support; and
(2) detecting the trace of digestion formed on the thin membrane by the action of protease wherein the protease substrate is selected from the group consisting of collagen, gelatin, proteoglycan, fibronectin, laminin, elastin and casein, wherein the hardening agent is selected from chromium salts; N-methylol compounds; dioxane derivatives; compounds exhibiting activities through the activation of carboxyl group; activated vinyl compounds; activated halogen compounds; mucohalogen acids; isoxazole compounds; dialdehyde starch; 2-chloro-6-hydroxytriazinylated gelatin.

2. A method for measuring protease according to Claim 1, wherein the steps (1) and (2) of Claim 1 form part of the following sequence of steps:
(1) bringing one of two or more continuous slices of a biological sample into contact with a thin membrane which comprises a protease substrate together with a hardening agent and is formed on a surface of a support;
(2) bringing the remaining slices into contact with a thin membrane which comprises a protease substrate a hardening agent, and a protease inhibitor and is formed on a surface of a support:
(3) detecting traces of digestion formed on the thin membranes by the action of protease; and
(4) comparing the trace of digestion on the thin membrane used in the step (1) with the trace of digestion on the thin membrane used in the step (2).

3. A method for measuring protease according to Claim 1, wherein the steps (1) and (2) of Claim 1 form part of the following sequence of steps:
(1) bringing one of two or more continuous slices of a biological sample into contact with a thin membrane which comprises a protease substrate together with a hardening agent and is formed on a surface of a support;
(2) bringing the remaining slices into contact with a thin membrane which comprises a protease substrate different from the protease substrate contained in the thin membrane used in the step (1) together with a hardening agent and is formed on a surface of a support;
(3) detecting traces of digestion formed on the thin membranes by the action of protease; and
(4) comparing the trace of digestion on the thin membrane used in the step (1) with the trace of digestion on the thin membrane used in the step (2).

4. A method for measuring protease, according to Claim 1, wherein the steps (1) and (2) of Claim 1 form part of the following sequence of steps:
(1) bringing a sample containing protease into contact with a thin membrane which comprises at least the following two layers: layer (a) which contains a protease substrate, a hardening agent, and a protease inhibitor and is formed on a surface of a support, and layer (b) which contains a protease substrate and a hardening agent and is laminated on the layer (a);
(2) detecting traces of digestion formed on the thin membrane by the action of protease; and
(3) comparing the trace of digestion on the layer (a) with the trace of digestion on the layer (b).

5. A method for measuring protease according to Claim 1, wherein the steps (1) and (2) of Claim 1 form part of the following sequence of steps:
(1) bringing a sample containing protease into contact with a thin membrane which comprises at least the following two layers: layer (a) which contains a protease substrate together with a hardening agent and is formed on a surface of a support and layer (b) which contains a protease substrate different from the protease substrate contained in the layer (a) together with a hardening agent and is laminated on the layer (a);
(2) detecting traces of digestion formed on the thin membrane by the action of protease: and
(3) comparing the trace of digestion on the layer (a) with the trace of digestion on the layer (b).

6. The method of any one of claims 1 to 5 wherein the sample is a biological sample isolated or collected from a patient.

7. The method of any one of claims 1 to 6 wherein the detection is performed by using a thin membrane containing one or more substances selected from the group consisting of metals, metal oxides, pigments and dyes and having a maximum transmission density of 0.01 or higher at a wavelength ranging from 400 nm to 700 nm.

8. The method of any one of claims 1 to 7 wherein the protease is matrix metalloproteinase.

9. A method of diagnosing a disease involving protease which comprises steps of:
(1) bringing a biological sample isolated or collected from a patient into contact with a thin membrane which comprises a protease substrate together with a hardening agent and is formed on a surface of a support; and
(2) detecting the trace of digestion formed on the thin membrane by the action of protease wherein the protease substrate is selected from the group consisting of collagen, gelatin, proteoglycan, fibronectin, laminin, elastin and casein, wherein the hardening agent is selected from chromium salts; N-methylol compounds; dioxane derivatives; compounds exhibiting activities through the activation of carboxyl group; activated vinyl compounds; activated halogen compounds; mucohalogen acids; isoxazole compounds; dialdehyde starch; 2-chloro-6-hydroxytriazinylated gelatin.

10. The method of 9 wherein the disease is selected from the group consisting of cancer, rheumatic diseases, periodontal diseases and alveolar pyorrhea.

## Patentansprüche

1. Verfahren zur Analyse von Protease, das die Schritte umfaßt:
(1) Inkontaktbringen einer Probe enthaltend Protease mit einer dünnen Membran, die ein Proteasesubstrat zusammen mit einem Härtungsmittel umfaßt und auf einer Oberfläche eines Trägers gebildet ist, und
(2) Detektieren von Zersetzungsspuren, die auf der dünnen Membran durch die Einwirkung der Protease gebildet werden,
wobei das Proteasesubstrat ausgewählt ist aus der Gruppe bestehend aus Kollagen, Gelatine, Proteoglycan, Fibronectin, Laminin, Elastin und Kasein, und wobei das Härtungsmittel ausgewählt ist aus Chromsalzen, N-Methylolverbindungen, Dioxanderivaten, Verbindungen, die durch Aktivierung einer Carboxylgruppe Aktivität zeigen, aktivierten Vinylverbindungen, aktivierten Halogenverbindungen, Mucohalogensäuren, Isoxazolverbindungen, Dialdehydstärke, 2-Chlor-6-hydroxytriazinylierter Gelatine.

2. Verfahren zur Analyse von Protease gemäß Anspruch 1, wobei die Schritte (1) und (2) gemäß Anspruch 1 Teil der folgenden Abfolge von Schritten sind:
(1) Inkontaktbringen von einer von zwei oder mehr fortlaufenden Schnitten einer biologischen Probe mit einer dünnen Membran, die ein Proteasesubstrat zusammen mit einem Härtungsmittel umfaßt und auf der Oberfläche eines Trägers gebildet ist,
(2) Inkontaktbringen der verbleibenden Schnitte mit einer dünnen Membran, die ein Proteasesubstrat, ein Härtungsmittel und einen Proteaseinhibitor umfaßt und auf einer Oberfläche eines Trägers gebildet ist,
(3) Detektieren der Zersetzungsspuren, die auf den dünnen Membranen durch die Einwirkung der Protease gebildet werden, und
(4) Vergleichen der Zersetzungsspuren auf der dünnen Membran, die in Schritt (1) verwendet wird, mit den Zersetzungsspuren auf der dünnen Membran, die in Schritt (2) verwendet wird.

3. Verfahren zur Analyse von Protease gemäß Anspruch 1, wobei die Schritte (1) und (2) gemäß Anspruch 1 Teil der Abfolge der folgenden Schritte sind:
(1) Inkontaktbringen einer von zwei oder mehr fortlaufenden Schnitten einer biologischen Probe mit einer dünnen Membran, die ein Proteasesubstrat zusammen mit einem Härtungsmittel umfaßt und auf einer Oberfläche eines Trägers gebildet ist,
(2) Inkontaktbringen der verbleibenden Schnitte mit einer dünnen Membran, die ein Proteasesubstrat, das von dem in der in Schritt (1) verwendeten dünnen Membran enthaltenden Proteasesubstrat verschieden ist, zusammen mit einem Härtungsmittel umfaßt und auf einer Oberfläche eines Trägers gebildet ist,
(3) Detektieren der Zersetzungsspuren, die auf den dünnen Membranen durch die Einwirkung der Protease gebildet werden, und
(4) Vergleichen der Zersetzungsspuren auf der dünnen Membran, die in Schritt (1) verwendet wird, mit den Zersetzungsspuren auf der dünnen Membran, die in Schritt (2) verwendet wird.

4. Verfahren zur Analyse von Protease gemäß Anspruch 1, wobei die Schritte (1) und (2) gemäß Anspruch 1 Teil der folgenden Abfolge von Schritten sind:
(1) Inkontaktbringen einer Probe enthaltend Protease mit einer dünnen Membran, die wenigstens die folgenden zwei Schichten umfaßt: Schicht (a), die ein Proteasesubstrat, ein Härtungsmittel und einen Proteaseinhibitor enthält und auf einer Oberfläche eines Trägers gebildet ist, und Schicht (b), die ein Proteasesubstrat und ein Härtungsmittel enthält und auf die Schicht (a) laminiert ist,
(2) Detektieren der Zersetzungsspuren, die auf der dünnen Membran durch die Einwirkung der Protease gebildet werden, und
(3) Vergleichen der Zersetzungsspuren auf der Schicht (a) mit den Zersetzungsspuren auf der Schicht (b).

5. Verfahren zur Analyse von Protease gemäß Anspruch 1, wobei die Schritte (1) und (2) gemäß Anspruch 1 Teil der folgenden Abfolge von Schritten sind:
(1) Inkontaktbringen einer Probe enthaltend Protease mit einer dünnen Membran, die wenigstens die folgenden zwei Schichten umfaßt: Schicht (a), die ein Proteasesubstrat zusammen mit einem Härtungsmittel enthält und auf einer Oberfläche eines Trägers gebildet ist, und Schicht (b), die ein Proteasesubstrat, das von dem in Schicht (a) enthaltenen Proteasesubstrat verschieden ist, zusammen mit einem Härtungsmittel enthält und auf die Schicht (a) laminiert ist,
(2) Detektieren der Zersetzungsspuren, die auf der dünnen Membran durch die Einwirkung der Protease gebildet werden, und
(3) Vergleichen der Zersetzungsspuren auf der Schicht (a) mit den Zersetzungsspuren auf der Schicht (b).

6. Verfahren gemäß einem der Ansprüche 1 bis 5, wobei die Probe eine biologische Probe ist, die aus einem Patienten isoliert oder entnommen wurde.

7. Verfahren gemäß einem der Ansprüche 1 bis 6, wobei die Detektion durch Verwendung einer dünnen Membran durchgeführt wird, die eine oder mehrere Substanzen ausgewählt aus der Gruppe bestehend aus Metallen, Metalloxiden, Pigmenten und Farbstoffen enthält und die eine maximale Transmissionsdichte von 0,01 oder höher bei einer Wellenlänge im Bereich von 400 bis 700 nm aufweist.

8. Verfahren gemäß einem der Ansprüche 1 bis 7, wobei die Protease Matrixmetalloproteinase ist.

9. Verfahren zum Diagnostizieren einer mit Protease verbundenen Krankheit, das die Schritte umfaßt:
(1) Inkontaktbringen einer biologischen Probe, die aus einem Patienten entnommen oder isoliert wurde, mit einer dünnen Membran, die ein Proteasesubstrat zusammen mit einem Härtungsmittel umfaßt und auf einer Oberfläche eines Trägers gebildet ist, und
(2) Detektieren der Zersetzungsspuren, die auf der dünnen Membran durch die Einwirkung der Protease gebildet werden,
wobei das Proteasesubstrat ausgewählt ist aus der Gruppe bestehend aus Kollagen, Gelatine, Proteoglycan, Fibronectin, Laminin, Elastin und Kasein, und wobei das Härtungsmittel ausgewählt ist aus Chromsalzen, N-Methylolverbindungen, Dioxanderivaten, Verbindungen, die durch Aktivierung einer Carboxylgruppe Aktivität zeigen, aktivierten Vinylverbindungen, aktivierten Halogenverbindungen, Mucohalogensäuren, Isoxazolverbindungen, Dialdehydstärke, 2-Chlor-6-hydroxytriazinylierter Gelatine.

10. Verfahren gemäß Anspruch 9, wobei die Krankheit ausgewählt ist aus der Gruppe bestehend aus Krebs, rheumatischen Erkrankungen, Parodontalerkrankungen und alveolarem Eiterfluß.

## Revendications

1. Une methode pour mesurer une protéase qui comprend les étapes de :
(1) mettre en contact un échantillon contenant la protéase avec une couche mince qui comprend un substrat de protéase en mélange avec un agent de réticulation et qui est formée sur une surface d'un support ; et
(2) détecter la trace de digestion formée sur la menbrane mince par l'action de protéase, dans laquelle le substrat de protéase est sélectionné parmi le groupe constitué de collagène, de gélatine, de protéoglycanne, de fibronectine, de laminine, d'élastine et de caséine, dans laquelle l'agent de réticulation est sélectionné parmi les sels de chrome, les composés N-methylole, les dérivés de dioxane, les composés exhibants leurs activités par l'activation du groupe carbonyle; les composés de vinyl activés ; les composés halogénés activés ; les acides mucohalogène; les composés isoxazole; l'amidon dialdéhyde ; la gélatine 2-chloro-6-hydroxytriazinylée.

2. Une methode pour mesurer une protéase selon la revendication 1, dans laquelle les étapes (1) et (2) de la revendication 1 font partie de la séquence d'étapes suivante :
(1) mettre en contact l'une de deux ou davantage de tranches continues d'un échantillon biologique avec une membrane mince qui comprend un substrat de protéase en mélange avec un agent de réticulation et qui est formée sur une surface d'un support ;
(2) mettre en contact les tranches restantes avec une membrane mince qui comprend un substrat de protéase, un agent de réticulation et un inhibiteur de protéase et qui est formée sur une surface d'un support ;
(3) détecter les traces de digestion formée sur les membranes minces par l'action de protéase ; et
(4) comparer la trace de digestion sur la membrane mince utilisée dans l'étape (1) avec la trace de digestion sur la membrane mince utilisée dans l'étape (2).

3. Une methode pour mesurer une protéase selon la revendication 1, dans laquelle les étapes (1) et (2) de la revendication 1 font partie de la séquence d'étapes suivante :
(1) mettre en contact l'une de deux ou davantage de tranches continues d'un échantillon biologique avec une membrane mince qui comprend un substrat de protéase en mélange avec un agent de réticulation et qui est formée sur une surface d'un support ;
(2) mettre en contact les tranches restantes avec une membrane mince qui comprend un substrat de protéase différent du substrat de protéase contenu dans la membrane mince utilisé dan l'étape (1) en mélange avec un agent de réticulation et qui est formée sur une surface d'un support ;
(3) détecter les traces de digestion formée sur les membranes minces par l'action de protéase ; et
(4) comparer la trace de digestion sur la membrane mince utilisée dans l'étape (1) avec la trace de digestion sur la membrane mince utilisée dans l'étape (2).

4. Une methode pour mesurer une protéase selon la revendication 1, dans laquelle les étapes (1) et (2) de la revendication 1 font partie de la séquence d'étapes suivante :
(1) mettre en contact un échantillon contenant la protéase avec une membrane mince qui comprend au moins les deux couches suivantes : couche (a) qui contient un substrat de protéase, un agent de réticulation et un inhibiteur de protéase et qui est formée sur une surface d'un support et couche (b) qui contient un substrat de protéase et un agent de réticulation et qui est laminée sur la couche (a) ;
(2) détecter les traces de digestion formée sur les membranes minces par l'action de protéase ; et
(3) comparer la trace de digestion sur la couche (a) avec la trace de digestion sur la couche (b).

5. Une methode pour mesurer une protéase selon la revendication 1, dans laquelle les étapes (1) et (2) de la revendication 1 font partie de la séquence d'étapes suivante :
(1) mettre en contact un échantillon contenant la protéase avec une membrane mince qui comprend au moins les deux couches suivantes : couche (a) qui contient un substrat de protéase, en mélange avec un inhibiteur de protéase et qui est formée sur une surface d'un support et couche (b) qui contient un substrat de protéase différent du substrat de protéase contenu dans la couche (a) en mélange avec un agent de réticulation et qui est laminée sur la couche (a) ;
(2) détecter les traces de digestion formée sur les membranes minces par l'action de protéase ; et
(3) comparer la trace de digestion sur la couche (a) avec la trace de digestion sur la couche (b).

6. La méthode de l'une quelconque des revendications 1 à 5, dans laquelle l'échantillon est un échantillon biologique isolé ou recueilli à partir d'un patient.

7. La méthode de l'une quelconque des revendications 1 à 6, dans laquelle la détection est réalisée en utilisant une membrane mince contenant une ou plusieurs substances sélectionnées parmis le groupe constitué de métaux et d'oxides de métaux, de pigments et de matières colorantes et ayant une densité de transmission maximale de 0.01 ou davantage à une longueur d'onde dans la gamme de 400 nm à 700 nm.

8. La méthode de l'une quelconque des revendications 1 à 7, dans laquelle la protéase est une métalloprotéinase de matrice.

9. Une methode pour diagnostiquer une maladie implicant une protéase qui comprend les étapes de :
(1) mettre en contact un échantillon biologique isolé ou recueilli à partir d'un patient avec une couche mince qui comprend un substrat de protéase en mélange avec un agent de réticulation et qui est formée sur une surface d'un support ; et
(2) détecter la trace de digestion formée sur la menbrane mince par l'action de protéase, dans laquelle le substrat de protéase est sélectionné parmi le groupe constitué de collagène, de gélatine, de protéoglycanne, de fibronectine, de laminine, d'élastine et de caséine, dans laquelle l'agent de réticulation est sélectionné parmi les sels de chrome, les composés N-methylole, les dérivés de dioxane, les composés exhibants leurs activités par l'activation du groupe carbonyle; les composés de vinyl activés ; les composés halogénés activés ; les acides mucohalogène; les composés isoxazole; l'amidon dialdéhyde ; la gélatine 2-chloro-6-hydroxytriazinylée.

10. La méthode selon 9, dans laquelle la maladie est sélectionnée parmi le groupe constitué du cancer, des maladies rheumatiques, des maladies périodontales et pyorrhea alveolar.
